# EUROPÄISCHE PATENTSCHRIFT

(11) **EP 3 500 110 B1**
(45) Veröffentlichungstag und Bekanntmachung des Hinweises auf die Patenterteilung: **02.10.2024**
(21) Anmeldenummer: 17761195.1
(22) Anmeldetag: 17.08.2017
(51) Int. Cl.: A23L 3/26, A01C 1/08, A61L 2/08, B65B 55/08, G21K 5/08, H01J 33/00, B07B 13/11, B07C 5/00, B65B 55/16, B65B 37/04, B07C 5/04, B07C 5/342

(54) **VORRICHTUNGEN UND VERFAHREN ZUM PASTEURISIEREN UND/ODER STERILISIEREN VON PARTIKELFÖRMIGEM GUT**
DEVICES AND METHOD FOR PASTEURISING AND/OR STERILISING PARTICULATE MATERIAL
DISPOSITIFS ET PROCEDE DE PASTEURISATION ET/OU DE STERILISATION DE PRODUITS PARTICULAIRES

(30) Priorität: 20.08.2016 EP 16185055
(43) Veröffentlichungstag der Anmeldung: 26.06.2019
(73) Patentinhaber: Bühler AG, 9240 Uzwil (CH)
(72) Erfinder: HERSCHE, Martin, 9230 Flawil (CH); MENESES, Nicolas, 9200 Gossau (CH); CURRIE, Alasdair, London N7 8QB (GB); SCHÖNENBERGER, Niklaus, 9100 Herisau (CH); SCHEIWILLER, Thomas, 9524 Zuzwil (CH)
(74) Vertreter: Hepp Wenger Ryffel AG
(86) Internationale Anmeldenummer: PCT/EP2017/070842
(87) Internationale Veröffentlichungsnummer: WO 2018/036899

(56) Entgegenhaltungen:
- EP-A1- 0 769 890
- EP-A2- 0 104 938
- DE-A1- 102012 209 434
- DE-A1- 3 816 946
- GB-A- 2 416 533
- JP-A- H11 101 900
- US-A- 4 734 586
- US-B1- 6 724 003
- US-B1- 6 734 383

## Beschreibung

Die vorliegende Erfindung betrifft Vorrichtungen und Verfahren zum Pasteurisieren und/oder Sterilisieren von partikelförmigem Gut mit Hilfe eines Elektronenstrahls.

Als partikelförmig werden hier und im Folgenden unter anderem aus Körnern und/oder Flocken bestehende Güter bezeichnet, wobei die Partikel beispielsweise eine kugelförmige, plattenförmige oder kantige Form haben können. Es kann sich auch um gemahlene Partikel handeln. Durch die Pasteurisierung und/oder Sterilisierung können beispielsweise Mikroorganismen zumindest grösstenteils abgetötet oder unschädlich gemacht werden. Insbesondere kann eine Reduktion von schädigenden Mikroorganismen um mindestens eine, bevorzugt mindestens fünf, besonders bevorzugt mindestens sieben Grössenordnungen erreicht werden.

Eine gattungsgemässe Vorrichtung ist beispielsweise aus der EP 1 080 623 B1 bekannt. Diese Vorrichtung enthält Vibrationsförderer, mit denen Saatgut zu einem transparenten Vorhang vereinzelt werden kann. Dieser Vorhang wird dann durch ein Elektronenfeld geführt, das von einem Elektronenbeschleuniger erzeugt wird und beispielsweise eine Sterilisierung des Saatguts bewirken kann. Um das Saatgut von einem Austrittsfenster des Elektronenbeschleunigers fernzuhalten, wird ein Gitter eingesetzt. Dieses Gitter sorgt jedoch insbesondere bei Gut aus feinen Partikeln für keinen ausreichenden Schutz des Austrittsfensters. Zudem wird ein Teil der Elektronen vom Saatgutstrom weggestreut und kann somit seine eigentliche Wirkung nicht entfalten. Weiterhin kann das Gitter nur schwer gereinigt werden und durch die Einwirkung des Elektronenstrahls leicht zerstört werden, weshalb es nur eine kurze Standzeit hat.

Aus der US 5,801,387 A ist eine weitere gattungsgemässe Vorrichtung bekannt. In der dortigen erfindungsgemässen Ausführung wird ein partikelförmiges Gut mit einem Vibrationsförderer in einen horizontalen Luftstrom eindosiert und dann einem Elektronenstrahl ausgesetzt. Anschliessend wird mit Hilfe einer Vakuumpumpe und eines Filters eine Klassierung vollzogen.

Weiterhin offenbart die DE 10 2012 209 434 A1 eine Vorrichtung, die ein rieselfähiges Produkt mit Hilfe einer Vibrationsfördereinrichtung und einer rotierenden Bürstenwalze vereinzelt und in Rotationen versetzt. Anschliessend passieren die Partikel frei fallend ein Elektronenfeld. Dieser Aufbau mit rotierender Bürstenwalze ist jedoch baulich aufwendig und störungsanfällig. Zudem erlauben die Vibrationsfördereinrichtung und die Bürstenwalze nicht immer eine zufriedenstellende Vereinzelung. Weiterhin ist die Bürstenwalze unter hygienischen Gesichtspunkten nachteilig, da sie nur umständlich von Staub gereinigt werden kann, der sich beim Betrieb der Vorrichtung ablagert. Zudem kann es passieren, dass bei einem Gut mit einer breiten Grössenverteilung der Partikel nicht alle Partikel mit der Bürstenwalze in Kontakt geraten oder die Partikel verschieden beschleunigt werden. Ausserdem kann die Bürstenwalze Turbulenzen im Gut hervorrufen, wodurch einzelne Partikel auf die Elektronenquelle treffen und diese verschmutzen oder sogar beschädigen können.

In der EP 0 513 135 B1 ist eine Vorrichtung offenbart, mit der Saatgut mittels Zellradschleusen in einen vertikalen Fallschacht eingeleitet wird, wo es im senkrechten Fall von Elektronenstrahlen beaufschlagt wird. Auch diese Zellradschleusen lassen jedoch nicht immer eine zufriedenstellende Vereinzelung zu. Zudem findet die Behandlung in einem Vakuum statt, wodurch die Vorrichtung apparativ aufwendig und störungsanfällig ist und zudem hohe Betriebskosten verursacht. Zudem ist die Geschwindigkeit des Guts durch den Abstand der oberen Zellradschleuse von der Elektronenquelle festgelegt und kann nicht variiert werden.

Aus der EP 0 705 531 B1 ist eine weitere Vorrichtung bekannt, die das Saatgut mittels einer nicht näher beschriebenen Dosiereinrichtung in eine Prozesskammer eingeleitet wird, in der es senkrecht durch einen Elektronenstrahl fällt. Auch hiermit kann jedoch keine ausreichende Vereinzelung erreicht werden.

Die in US 6,486,481 B1 offenbarte Vorrichtung enthält einen Rütteltisch, auf dem ein polymeres Material bewegt und einem Elektronenstrahl ausgesetzt wird. Dies erfolgt jedoch nicht zur Pasteurisierung oder Sterilisierung, sondern zur Reduktion des Molekulargewichts des polymeren Materials.

Das Dokument US 6,724,003 B1 offenbart eine Elektronenstrahl-Bestrahlungsreaktionsvorrichtung zur Bestrahlung eines gewünschten zu behandelnden Objekts, wie z. B. eines Abgases, mit einem Elektronenstrahl. Ein flexibles hermetisches Dichtungselement ist zwischen einem Abschnitt um einen Endabschnitt einer Abtaströhre der Vorrichtung und einer peripheren Kante eines Elektronenstrahl-Empfangsfensters in einer Seitenwand eines Elektronenstrahls Reaktionsvorrichtung vorgesehen.

In EP 0 769 890 A1 sind Verfahren und Vorrichtungen zum Kühlen von Fensterfolien zum Extrahieren von Elektronenstrahlen aus einem Elektronenstrahlbeschleuniger des Abtasttyps offenbart. Der Beschleuniger enthält eine primäre Fensterfolie des Doppelfenstertyps und eine sekundäre Fensterfolie. Kühlgase werden von beiden Seiten gegen eine Elektronenstrahlabtastfläche geblasen, um die primäre Fensterfolie zu kühlen. Dann wird der Fluss der Kühlgase in der Mitte der primären Fensterfolie umgekehrt wird, und die Kühlgase zirkulieren durch Ansaugen der Kühlgase, um dadurch gleichzeitig die sekundäre Fensterfolie zu kühlen.

Es ist daher eine Aufgabe der vorliegenden Erfindung, die aus dem Stand der Technik bekannten Nachteile zu überwinden. Insbesondere sollen Vorrichtungen und Verfahren bereitgestellt werden, mit denen partikelförmiges Gut effektiv, zuverlässig, mit hohem Durchsatz und möglichst einfach pasteurisiert und/ oder sterilisiert werden kann. Insbesondere soll durch den Elektronenstrahl entstehendes Ozon möglichst effektiv und zuverlässig abgeführt werden. Ausserdem soll bevorzugt die Vorrichtung, insbesondere ein Austrittsfenster einer Elektronenquelle, vor Beschädigungen durch das Saatgut geschützt werden und leicht gereinigt werden können, und auch die Mittel zum Vereinzeln sollten leicht gereinigt werden können.

Diese und weitere Aufgaben werden gelöst durch die erfindungsgemässe Vorrichtung zum Pasteurisieren und/oder Sterilisieren von partikelförmigem Gut, deren Merkmale und Vorteile weiter unten im Detail erläutert werden.

Zunächst werden einige optionale Merkmale der erfindungsgemässen Vorrichtung dargestellt:
Die Vorrichtung kann eine erste Vibrationsfläche, mindestens eine Elektronenquelle zum Erzeugen eines Elektronenstrahls und eine stromabwärts von der ersten Vibrationsfläche angeordnete Behandlungszone enthalten. Hier und im Folgenden beziehen sich die Begriffe "stromabwärts" und "stromaufwärts" auf die Strömungsrichtung des partikelförmigen Guts bei bestimmungsgemässer Bedienung der Vorrichtung. Folglich wird eine erste Einheit als stromabwärts von einer zweiten Einheit bezeichnet, wenn sie bei bestimmungsgemässer Bedienung der Vorrichtung vom Gut nach der zweiten Einheit passiert wird. Analog wird eine erste Einheit als stromaufwärts von einer zweiten Einheit bezeichnet, wenn sie bei bestimmungsgemässer Bedienung der Vorrichtung vom Gut vor der zweiten Einheit passiert wird.

Die erste Vibrationsfläche ist bevorzugt im Wesentlichen horizontal ausgerichtet. Hier und im Folgenden wird unter einer (im Wesentlichen) horizontalen Ausrichtung stets eine (im Wesentlichen) horizontale Ausrichtung bei bestimmungsgemässer Installation der Vorrichtung verstanden. Sie ist zu Vibrationen anregbar, um das Gut zu fördern und zu vereinzeln. In der Behandlungszone ist das Gut mittels des Elektronenstrahls pasteurisierbar und/oder sterilisierbar.

Die erste Vibrationsfläche kann eine Vielzahl von Rinnen aufweisen, in denen das Gut förderbar und mittels deren es vereinzelbar ist. Die Rinnen können in einer Schnittebene senkrecht zur Strömungsrichtung ein im Prinzip beliebiges Profil aufweisen, sofern das Gut darin förderbar und vereinzelbar ist. Beispielsweise können sie ein bogenförmiges oder ein eckiges Profil aufweisen. Das Profil einer Rinne kann einen geraden mittleren Abschnitt und zwei sich vom mittleren Abschnitt schräg nach oben erstreckende seitliche Abschnitte aufweisen. Die seitlichen Abschnitte benachbarter Rinnen können sich an einer Kante treffen.

Vorzugsweise verläuft bzw. verlaufen mindestens eine Rinne, bevorzugt sämtliche Rinnen, im Wesentlichen parallel zur Strömungsrichtung des Guts. Mindestens eine Rinne, bevorzugt sämtliche Rinnen, kann/können eine Breite aufweisen, die im Bereich von 0,1 mm bis 40 mm, bevorzugt von 0,5 mm bis 30 mm, besonders bevorzugt von 1 mm bis 20 mm liegt.

In jeder der Rinnen können Partikel des partikelförmigen Guts gefördert und vereinzelt werden. Die Vereinzelung kann dabei bereits dadurch stattfinden, dass die Partikel des partikelförmigen Guts auf die Rinnen aufgeteilt werden.

Bevorzugt ist es, wenn die erste Vibrationsfläche derart ausgebildet und angeordnet und zu Vibrationen anregbar und/oder eine allfällige nachfolgend beschriebene stromabwärts angeordnete Umlenkfläche und/oder eine allfällige nachfolgend beschriebene stromabwärts angeordnete Rutschfläche derart angeordnet und ausgebildet ist/sind, dass das Gut frei fallend pasteurisierbar und/oder sterilisierbar ist. Das Gut wird dabei als "frei fallend" bezeichnet, wenn die Flugbahnen der einzelnen Partikel des Guts allein durch ihre Geschwindigkeit, die auf sie einwirkende Schwerkraft und gegebenenfalls ein Prozessgas, von dem das Gut umgeben ist, bestimmt werden Insbesondere rutschen die Partikel des Guts nicht auf einer Fläche durch die Behandlungszone. Das Prozessgas kann beispielsweise Luft sein. Es ist jedoch auch denkbar, dass als Prozessgas ein Gas eingesetzt wird, welches eine Ozonbildung verhindert, wie beispielsweise Stickstoff.

Mittels des Elektronenstrahls kann nicht nur das Gut selbst behandelt werden, sondern auch allfälliges das Gut umgebendes Prozessgas und/oder weitere mit dem Gut strömende Partikel wie beispielsweise Staub.

Mit Vorteil weist die Vorrichtung stromabwärts von der ersten Vibrationsfläche und stromaufwärts von der Behandlungszone eine geneigte Rutschfläche auf, welche derart ausgebildet und angeordnet ist, dass das Gut darauf in Richtung der Behandlungszone rutschen kann. Aufgrund der beim Rutschen entstehenden Beschleunigung werden die Partikel des Guts weiter vereinzelt. Zudem kann das Gut in definierter Weise, insbesondere mit definierter Geschwindigkeit und Bahnkurve, der Behandlungszone zugeführt werden. Insbesondere kann durch die Wahl der Länge und des Neigungswinkels der Rutsche die Geschwindigkeit des Guts nach dem Verlassen der Rutsche eingestellt werden.

Bevorzugt weist die Rutschfläche mindestens eine Rinne und bevorzugt eine Vielzahl von Rinnen auf, welche derart ausgebildet und angeordnet ist/sind, dass das Gut darin rutschen und vereinzelt werden kann. Hierdurch können Geschwindigkeit und Bahnkurve der Partikel des Guts weiter definiert eingestellt werden. Mit besonderem Vorteil sind die Rinnen der Rutschfläche derart an die Rinnen der ersten Vibrationsfläche angepasst, dass von ein und derselben Rinne der ersten Vibrationsfläche auf die Rutschfläche gelangende Partikel in ein und dieselbe Rinne der Rutschfläche gelangen. Hierdurch kann eine turbulente Bewegung der Partikel verhindert werden, so wie sie beispielsweise durch die in DE 10 2012 209 434 A1 offenbarte Bürstenwalze entstehen können. In einigen Anwendungen kann zudem der Vorteil erzielt werden, dass eine Rotation der Partikel des Guts verhindert wird.

Wie sich herausgestellt hat, ist die Rutschfläche vorteilhafterweise bezüglich einer Horizontalen unter einem Winkel nach unten geneigt, der im Bereich von 45° bis 85°, bevorzugt von 55° bis 75°, besonders bevorzugt von 60° bis 70° liegt (bei bestimmungsgemässer Installation der Vorrichtung). Für viele Güter sind Winkel in diesem Bereich gross genug, dass die Partikel auf einer tolerablen Länge der Rutsche ausreichend beschleunigt werden, und klein genug, um ein Abheben von der Rutschfläche zu verhindern.

Die mindestens eine Rinne der Rutschfläche kann in einer Schnittebene senkrecht zur Strömungsrichtung ein im Prinzip beliebiges Profil aufweisen, sofern das Gut darin rutschen und vereinzelt werden kann. Beispielsweise kann sie ein bogenförmiges oder ein eckiges Profil aufweisen. Vorzugsweise verläuft die mindestens eine Rinne der Rutschfläche im Wesentlichen parallel zur Stromrichtung des Guts. Die mindestens eine Rinne der Rutschfläche kann eine Breite aufweisen, die im Bereich von 40 mm bis 3300 mm, bevorzugt von 200 mm bis 600 mm, besonders bevorzugt von 230 mm bis 400 mm liegt. Das Profil einer Rinne der Rutschfläche kann einen geraden mittleren Abschnitt und zwei sich vom mittleren Abschnitt schräg nach oben erstreckende seitliche Abschnitte aufweisen. Sind mehrere Rinnen vorhanden, so können sich die seitlichen Abschnitte benachbarter Rinnen an einer Kante treffen.

Für viele Güter hat es sich ausserdem als nützlich erwiesen, wenn die Vorrichtung stromabwärts von der ersten Vibrationsfläche und stromaufwärts von der Behandlungszone, insbesondere stromaufwärts von der Rutschfläche, eine Umlenkfläche aufweist, welche derart ausgebildet und angeordnet ist, dass das Gut darauf umgelenkt und von der ersten Vibrationsfläche zur Rutschfläche und/oder in Richtung der Behandlungszone rutschen kann. Insbesondere wenn eine wie oben beschriebene Rutschfläche vorhanden ist, können die Partikel des Guts allmählich und kontrolliert an die Neigung der Rutschfläche herangeführt werden. Besonders vorteilhaft ist es, wenn die Rutschfläche, insbesondere ihre mindestens eine Rinne, derart auf das Gut und die erste Vibrationsfläche abgestimmt ist, dass die Partikel auf einer Parabelbahn geführt werden, auf der sie auch allein aufgrund der Einwirkung der Schwerkraft fallen würden.

Mit besonderem Vorteil weist auch die Umlenkfläche mindestens eine Rinne, bevorzugt eine Vielzahl von Rinnen auf, welche derart ausgebildet und angeordnet ist/sind, dass das Gut darin rutschen kann. Dies hat hier ebenfalls den Effekt, dass die Partikel des Guts kontrolliert geführt werden können und keine Turbulenzen entstehen können sowie insbesondere eine Rotation der Partikel verhindert werden kann.

Die mindestens eine Rinne der Umlenkfläche kann in einer Schnittebene senkrecht zur Strömungsrichtung ein im Prinzip beliebiges Profil aufweisen, sofern das Gut darin rutschen kann. Beispielsweise kann sie ein bogenförmiges oder ein eckiges Profil aufweisen. Das Profil einer Rinne der Umlenkfläche kann einen geraden mittleren Abschnitt und zwei sich vom mittleren Abschnitt schräg nach oben erstreckende seitliche Abschnitte aufweisen. Sind mehrere Rinnen vorhanden, so können sich die seitlichen Abschnitte benachbarter Rinnen an einer Kante treffen. Vorzugsweise verläuft die mindestens eine Rinne der Umlenkfläche im Wesentlichen parallel zur Strömungsrichtung des Guts. Die mindestens eine Rinne der Umlenkfläche kann eine Breite aufweisen, die im Bereich von 0,1 mm bis 40 mm, bevorzugt von 0,5 mm bis 30 mm, besonders bevorzugt von 1 mm bis 20 mm liegt.

Mit besonderem Vorteil sind die Rinnen der Umlenkfläche derart an die Rinnen der ersten Vibrationsfläche angepasst, dass von ein und derselben Rinne der ersten Vibrationsfläche auf die Umlenkfläche gelangende Partikel in ein und dieselbe Rinne der Umlenkfläche gelangen. Ebenfalls mit besonderem Vorteil sind die Rinnen der Rutschfläche derart an die Rinnen der Umlenkfläche angepasst, dass von ein und derselben Rinne der Umlenkfläche auf die Umlenkfläche gelangende Partikel in ein und dieselbe Rinne der Rutschfläche gelangen.

In Abwandlung von den oben beschriebenen Ausführungsformen kann es jedoch auch für gewisse Güter sinnvoll sein, wenn die Rutschfläche und/oder die Umlenkfläche keine Rinnen aufweisen oder eine derartige Rutschfläche und/oder Umlenkfläche gar nicht vorhanden ist. Beispielsweise kann das Gut auch direkt von der ersten Vibrationsfläche zur Rutschfläche geführt werden, oder es kann direkt von der Umlenkfläche zur Behandlungszone geführt werden. Falls das Gut beispielsweise aus Flocken besteht, so kann eine Umlenkfläche die Flocken bremsen und anstauen. Um dies zu verhindern, kann es vorteilhaft sein, im Falle von Flocken auf eine Umlenkfläche zu verzichten. Wenn beispielsweise das Gut senkrecht durch die Behandlungszone fallen soll, kann auf die Umlenkfläche und auf die Rutschfläche verzichtet werden.

Stromaufwärts von der ersten Vibrationsfläche kann die Vorrichtung eine im Wesentlichen ebene und bevorzugt im Wesentlichen horizontal ausgerichtete zweite Vibrationsfläche aufweisen, welche zu Vibrationen anregbar ist. Mit Hilfe einer solchen zweiten Vibrationsfläche kann der Durchsatz des Guts kontrolliert werden, und es kann auch bereits eine Vorvereinzelung stattfinden.

Zweckmässigerweise wird beim Betrieb der Vorrichtung die zweite Vibrationsfläche zu Vibrationen mit einer zweiten Amplitude angeregt, die kleiner ist als eine erste Amplitude der Vibrationen, zu denen die stromabwärts angeordnete erste Vibrationsfläche angeregt wird. Auf diese Weise wird die Geschwindigkeit des Guts erhöht und eine weitere Vereinzelung erreicht.

Die Vibrationsfläche(n) und/oder die Umlenkfläche und/oder die Rutschfläche kann beispielsweise aus einem Metall bestehen.

Die mindestens eine Elektronenquelle kann an sich bekannt sein. Die Vorrichtung kann eine oder mehrere Elektronenquellen enthalten. Sind mehrere Elektronenquellen vorhanden, so können diese einander gegenüberliegend oder bezüglich der Strömungsrichtung des Guts nacheinander angeordnet sein.

Weiterhin ist es auch denkbar und liegt im Rahmen der Erfindung, dass die Vorrichtung mehrere erste Vibrationsflächen und/oder mehrere Behandlungszonen aufweist. Auf diese Weise kann eine noch effektivere Pasteurisierung und/oder Sterilisierung erreicht werden. Alternativ kann das Gut mehrmals durch ein und dieselbe Behandlungszone geführt werden.

Im Folgenden wird die erfindungsgemässe Vorrichtung im Detail erläutert:
Die Erfindung betrifft eine Vorrichtung zum Pasteurisieren und/oder Sterilisieren von partikelförmigem Gut, die ebenfalls mindestens eine Elektronenquelle zum Erzeugen eines Elektronenstrahls und eine Behandlungszone enthält, in der das Gut insbesondere frei fallend mittels des Elektronenstrahls pasteurisierbar und/oder sterilisierbar ist. Die Vorrichtung weist im Bereich der Behandlungszone einen Gutkanal auf, in dem das Gut mittels des Elektronenstrahls pasteurisierbar und/oder sterilisierbar ist. Sie weist ferner mindestens einen von einem Fluid durchströmbaren Nebenkanal auf, welcher zumindest teilweise zwischen der Elektronenquelle und dem Gutkanal verläuft und vom Gutkanalfluid getrennt ist.

Das durch den Nebenkanal strömende Fluid kann dazu eingesetzt werden, die Elektronenquelle und insbesondere ein Austrittsfenster der Elektronenquelle zu kühlen. Alternativ oder zusätzlich kann das Fluid dafür eingesetzt werden, aufgrund des Elektronenstrahls entstehendes Ozon abzuführen. Weder diese Kühlung noch diese Abführung von Ozon aus dem Nebenkanal haben einen Einfluss auf den Fluidstrom im Gutkanal. Unter einer Fluidtrennung wird verstanden, dass weder das Fluid vom Nebenkanal in den Gutkanal eintreten kann noch das Gut und allfälliges das Gut umgebendes Prozessgas vom Gutkanal in den Nebenkanal eintreten kann. Hierdurch wird eine Beschädigung oder Verschmutzung der Elektronenquelle, insbesondere eines Austrittsfensters Elektronenquelle, durch das Gut verhindert. Das Fluid kann eine Flüssigkeit oder ein Gas sein, wie beispielsweise Luft. Hierdurch werden die Nachteile von Gittern überwunden, so wie sie beispielsweise in EP 1 080 623 B1 beschrieben sind.

Zwischen der Elektronenquelle und dem Gutkanal ist eine für den Elektronenstrahl zumindest teilweise durchlässige Schutzfolie angeordnet, die den Gutkanal vom Nebenkanal trennt. Bevorzugt ist der Nebenkanal zumindest teilweise zwischen der Elektronenquelle und der Schutzfolie angeordnet; das sich darin befindende Fluid wird dort also beim Betrieb der Vorrichtung dem Elektronenstrahl ausgesetzt. Die Schutzfolie besteht vorzugsweise aus einem Metall, wie beispielsweise Titan, Aluminium, Gold, Silber oder Kupfer. Bei dem Metall kann es sich auch um eine Legierung handeln. In einigen Anwendungen kann die Schutzfolie beschichtet sein. Alternativ ist es auch denkbar und liegt im Rahmen der Erfindung, dass die Schutzfolie aus einem Kunststoff besteht.

Mit besonderem Vorteil enthält die Vorrichtung eine Kassettenaufnahme zum Aufnehmen einer Kassette, wobei die Kassette zumindest teilweise den Gutkanal und den mindestens einen Nebenkanal begrenzt und eine Folienaufnahme zur Aufnahme der Schutzfolie enthält. Weiterhin vorteilhaft ist die Elektronenquelle derart beweglich, insbesondere schwenkbar und/oder verschiebbar, relativ zur Kassettenaufnahme angeordnet, dass sie von der Kassette weg bewegbar ist. Dies vereinfacht den Zugang zur Kassette und damit auch zur Schutzfolie. Die Schutzfolie kann folglich leichter ausgetauscht werden, wenn sie vom Gut verschmutzt oder beschädigt wurde. Die Schutzfolie kann lösbar von der Folienaufnahme aufgenommen oder aufnehmbar sein. Erfindungsgemäss enthält die Vorrichtung eine Absaugeinrichtung zum Absaugen von allfälligem Prozessgas, das das Gut umgibt. Die Absaugeinrichtung zum Absaugen des Prozessgases ist stromabwärts von der Behandlungszone angeordnet, bevorzugt in einem Abstand von der Behandlungszone im Bereich von 50 mm bis 250 mm. Auf diese Weise kann mit dem Prozessgas besonders effektiv Ozon abgeführt werden, welches sich beim Durchtritt durch die Behandlungszone aufgrund der Beaufschlagung mit dem Elektronenstrahl gebildet hat.

Es ist auch denkbar, dass einige Güter in einem entgegen der Strömungsrichtung des Guts strömenden Prozessgas durch die Behandlungszone strömen und die Absaugeinrichtung zum Absaugen von Prozessgas stromaufwärts von der Behandlungszone angeordnet ist. Dies ist insbesondere vorteilhaft für schwerere Partikel, die weniger von einem das Gut umgebenden Prozessgas beeinflusst werden. Hierdurch kann erreicht werden, dass kein unbehandeltes Prozessgas in einen stromabwärts von der Behandlungszone angeordneten Reinbereich eindringen kann, in dem das Gut bei bestimmungsgemässem Betrieb der Vorrichtung pasteurisiert und/oder sterilisiert sein sollte. Somit kann eine Rekontamination verhindert werden.

Stromabwärts von der Behandlungszone kann die Vorrichtung ferner eine Sortiereinrichtung aufweisen, welche eine Messeinheit und eine Ausstosseinheit enthält. Die Messeinheit und die Ausstosseinheit sind derart ausgebildet, dass mittels der Ausstosseinheit einzelne Körner des Guts anhand mindestens einer mittels der Messeinheit gemessenen Eigenschaft der einzelnen Partikel ausstossbar sind. Derartige Sortiereinrichtungen sind an sich bekannt, beispielsweise aus WO 2006/010873 A1. Einerseits können sie dem Ausstossen von einzelnen Partikeln dienen, die eine vorgegebene Eigenschaft nicht erfüllen. Beispielsweise kann die Aussortierung aufgrund einer gemessenen Grösse oder Farbe erfolgen, die ausserhalb eines vorgegebenen Toleranzbereichs liegt. Andererseits kann die Messeinheit derart beschaffen sein, dass damit Überlappungen der einzelnen Partikel detektiert werden können. Eine solche Überlappung kann darauf hindeuten, dass nicht alle Partikel beim Durchtritt durch die Behandlungszone vom Elektronenstrahl ausreichend beaufschlagt wurden. Diese mehreren, sich überlappenden Partikel können dann zur Sicherheit mit Hilfe der Ausstosseinheit ausgestossen werden.

Ebenfalls vorteilhaft ist es, wenn die Vorrichtung mindestens eine stromabwärts von der Behandlungszone angeordnete Gasaustrittsöffnung zum Einblasen eines Reinigungsgases auf das Gut aufweist. Auf diese Weise kann übriges Ozon abtransportiert werden. Alternativ kann das Reinigungsgas auch stromaufwärts von der Behandlungszone eingeleitet werden, insbesondere im Falle von schwereren Partikeln, der Flugbahn weniger vom Reinigungsgasstrom beeinflusst wird.

Als nicht zur Erfindung gehörig offenbart wird ferner eine Kassette zum Einsetzen in eine Kassettenaufnahme einer Vorrichtung zum Pasteurisieren und/oder Sterilisieren von partikelförmigem Gut, die mindestens eine Elektronenquelle zum Erzeugen eines Elektronenstrahls enthält. Insbesondere kann es sich um eine wie oben beschriebene Vorrichtung handeln. Die Kassette enthält Begrenzungsflächen zum zumindest teilweisen Begrenzen eines Gutkanals und mindestens eines Nebenkanals der Vorrichtung sowie eine Folienaufnahme zur Aufnahme einer für den Elektronenstrahl zumindest teilweise durchlässige Schutzfolie. Die Kassette ist derart in die Kassettenaufnahme einsetzbar, dass die Vorrichtung im Bereich einer Behandlungszone einen Gutkanal aufweist, in dem das Gut mittels des Elektronenstrahls pasteurisierbar und/oder sterilisierbar ist, und dass die Vorrichtung mindestens einen von einem Fluid durchströmbaren Nebenkanal aufweist, welcher zumindest teilweise zwischen der Elektronenquelle und dem Gutkanal verläuft und vom Gutkanal fluidgetrennt ist. Die Begrenzungsflächen der Kassette begrenzen dann zumindest teilweise den Gutkanal und den mindestens einen Nebenkanal.

Die erfindungsgemässe Vorrichtung kann eine Kassettenaufnahme und eine darin eingesetzte Kassette, insbesondere eine wie oben beschriebenen Kassette, aufweisen. Die Kassette kann zumindest teilweise den Gutkanal und den mindestens einen Nebenkanal begrenzen und eine Folienaufnahme zur Aufnahme der Schutzfolie enthalten. Die Schutzfolie kann von der Folienaufnahme aufgenommen sein. Die Elektronenquelle kann derart beweglich, insbesondere schwenkbar und/oder verschiebbar, relativ zur Kassettenaufnahme angeordnet sein, dass sie von der Kassette weg bewegbar ist.

In einem weiteren Aspekt betrifft die Erfindung auch ein Verfahren zum Pasteurisieren und/oder Sterilisieren von partikelförmigem Gut. Dieses Verfahren kann insbesondere mit einer wie oben beschriebenen Vorrichtung durchgeführt werden. Das Verfahren kann die folgenden Schritte enthalten, wenn die Vorrichtung eine wie oben beschriebene Vibrationsfläche aufweist:
a) Fördern und Vereinzeln des Guts mittels einer bevorzugt im Wesentlichen horizontal ausgerichteten ersten Vibrationsfläche, welche zu Vibrationen angeregt wird und eine Vielzahl von Rinnen aufweist, in denen das Gut gefördert und mittels deren es vereinzelt wird,
b) Erzeugen eines Elektronenstrahls,
c) Pasteurisieren und/oder Sterilisieren des insbesondere frei fallenden Guts mittels des Elektronenstrahls in einer Behandlungszone.

Mit diesen Verfahren können die bereits oben erläuterten Vorteile erzielt werden.

Das erfindungsgemässe Verfahren zum Pasteurisieren und/oder Sterilisieren von partikelförmigem Gut enthält die folgenden Schritte:
b) Erzeugen eines Elektronenstrahls,
c) Pasteurisieren und/oder Sterilisieren des insbesondere frei fallenden Guts mittels des Elektronenstrahls in einer Behandlungszone.

Dabei strömt das Gut im Bereich der Behandlungszone durch einen Gutkanal, in dem es mittels des Elektronenstrahls pasteurisiert und/oder sterilisiert wird. Ein Fluid strömt durch mindestens einen Nebenkanal, welcher zumindest teilweise zwischen der Elektronenquelle und dem Gutkanal verläuft und vom Gutkanal fluidgetrennt ist. Wie bereits ausgeführt wurde, kann mit diesem Fluid, welches eine Flüssigkeit oder ein Gas sein kann, Ozon abgeführt werden, welches auf Grund des Elektronenstrahls entstanden ist. Alternativ oder zusätzlich kann das Fluid auch zum Kühlen der Elektronenquelle, insbesondere eines Austrittsfensters, eingesetzt werden. Das Fluid kann den Nebenkanal parallel oder entgegengesetzt zur Strömungsrichtung des Guts durchströmen. Als Fluid kann ein Gas eingesetzt wird, welches eine Ozonbildung verhindert, wie beispielsweise Stickstoff. Das durch den Nebenkanal strömende Fluid kann mit dem im Gutkanal strömenden Prozessgas identisch sein oder davon verschieden sein. Andere Strömungsrichtungen des Fluids sind jedoch ebenso denkbar und liegen im Rahmen der Erfindung.

Für viele Güter, insbesondere für eine Vielzahl von Gewürzen, hat es sich als vorteilhaft erwiesen, wenn sich das Gut mit einer Geschwindigkeit durch die Behandlungszone bewegt, die im Bereich von 1 m/s bis 5 m/s, bevorzugt von 2 m/s bis 4 m/s, besonders bevorzugt von 2 m/s bis 3 m/s liegt. Diese Geschwindigkeit kann beispielsweise durch die Länge und den Neigungswinkel einer stromaufwärts angeordneten Rutschfläche eingestellt werden. Je höher die Geschwindigkeit des Guts ist, desto grösser ist der erreichbare Durchsatz. Beim freien Fall ist die Geschwindigkeit unabhängig vom Durchsatz, so dass beispielsweise Durchsätze im Bereich 100 kg/h bis 1000 kg/h bei der gleichen Geschwindigkeit erreicht werden können. Der Durchsatz hängt lediglich von der Vibration der Vibrationsfläche(n) und der Dimensionen und Orientierungen der allfälligen Umlenk- und Rutschfläche ab. Zudem sinkt mit steigender Geschwindigkeit des Guts die Wahrscheinlichkeit von Kollisionen der Partikel mit der Elektronenquelle oder der Schutzfolie. Andererseits dürfen die Geschwindigkeiten aber auch nicht zu gross gewählt werden, damit das Gut ausreichend lange im Elektronenstrahl verbleibt, um pasteurisiert und/oder sterilisiert zu werden.

Die Elektronen des Elektronenstrahls sollten bevorzugt eine Energie aufweisen, die im Bereich von 80 keV bis 300 keV, bevorzugt von 140 keV bis 280 keV, besonders bevorzugt von 180 keV bis 260 keV liegt. Geringere Elektronenenergien würden keine ausreichende Pasteurisierung und/oder Sterilisierung erzeugen. Durch höhere Elektronenenergien liessen sich keine wesentlich höheren Grade der Pasteurisierung und/oder Sterilisierung erreichen.

Vorteilhafterweise wird das Gut dem Elektronenstrahl für eine Behandlungszeit ausgesetzt, die im Bereich von 5 ms bis 25 ms liegt. Denn für eine ausreichende Pasteurisierung und/oder Sterilisierung ist eine gewisse minimale Behandlungszeit nötig. Zu lange Behandlungszeiten haben keinen nennenswert erhöhten Grad der Pasteurisierung und/oder Sterilisierung gezeigt und würden zudem den Durchsatz verringern.

Ebenfalls mit Vorteil wird das Gut mittels des Elektronenstrahls einer Strahlendosis ausgesetzt, die im Bereich von 1 kGy bis 45 kGy, bevorzugt von 8 kGy bis 30kGy, besonders bevorzugt von 10 kGy bis 16 kGy liegt.

Die Elektronenstromdichte in der Behandlungszone liegt bevorzugt im Bereich von 10¹⁵ s⁻¹·cm⁻² bis 2,77·10¹⁵ s⁻¹·cm⁻².

Erfindungsgemäss wird Prozessgas, welches das Gut umgibt, nach der Pasteurisierung und/oder Sterilisierung abgesaugt. Hierdurch kann nämlich bei der Pasteurisierung und/oder Sterilisierung entstehendes Ozon abgeführt werden.

Bei dem Gut kann es sich um ein Lebensmittel handeln, wie beispielsweise Getreide wie etwa Soja, Frühstückscerealien, Snacks, Nüsse wie etwa getrocknete Kokosnüsse, Mandeln, Erdnussbutter, Kakaobohnen, Schokolade, Schokoladenflüssigkeit, Schokoladenpulver, Schokoladenchips, Kakaoprodukte, Hülsenfrüchte, Kaffee, Samen wie etwa Kürbissamen, Gewürze (wie beispielsweise Kurkuma, insbesondere in Scheiben), Teemischungen, getrocknete Früchte, Pistazien, trockene Proteinprodukte, Bäckereiprodukte, Zucker, Kartoffelprodukte, Teigwaren, Babynahrung, getrocknete Eiprodukte, Sojaprodukte wie beispielsweise Sojabohnen, Verdickungsmittel, Hefen, Hefeextrakte, Gelatine oder Enzyme handeln.

Alternativ kann das Gut auch ein Tiernahrungsmittel sein, wie beispielsweise Pellets, Futter für Wiederkäuer, Geflügel, Wassertiere (insbesondere Fische) oder Haustiere, oder Mischfutter.

Es ist jedoch ebenso denkbar und liegt im Rahmen der Erfindung, dass das Gut beispielsweise ein Kunststoff wie etwa PET ist, beispielsweise in Form von Flocken oder Pellets.

Die Vorrichtung kann stromabwärts von der Behandlungszone und stromabwärts von der Absaugeinrichtung und/oder der Sortiereinrichtung und/oder der Gasaustrittsöffnung einen Reinbereich aufweisen, in dem das Gut bei bestimmungsgemässem Betrieb der Vorrichtung pasteurisiert und/oder sterilisiert ist.

Gelegentlich können in einer Elektronenquelle elektrische Durchschläge entstehen, die die Elektronenquelle beschädigen können. Um dem entgegenzuwirken, kann die Elektronenquelle derart ausgebildet sein, dass sie sich im Falle eines Durchschlags selbst ausschaltet. Dies hat jedoch zur Folge, dass das Gut dann weder pasteurisiert noch sterilisiert in den Reinbereich gelangt, und zwar möglicherweise sogar unbemerkt.

Um dies zu verhindern, kann die Vorrichtung mindestens einen Strahlungssensor enthalten, mit dessen Hilfe die Intensität des Elektronenstrahls detektiert werden kann. Derartige Strahlungssensoren für Elektronenstrahlen sind beispielsweise aus der US 6,657,212 oder der US 7,592,613 bekannt. Falls die gemessene Intensität einen vorgebbaren Schwellenwert unterschreitet (was auch ein Indiz für einen elektrischen Durchschlag sein kann), kann die Zuführung des Guts gestoppt werden. Alternativ oder zusätzlich kann das Gut ausgeleitet werden, um gegebenenfalls zu einem späteren Zeitpunkt einer Pasteurisierung und/oder Sterilisierung unterzogen zu werden. Zu diesem Zweck kann die Vorrichtung einen weiteren Gutauslass aufweisen, aus dem das zu einem späteren Zeitpunkt zu pasteurisierende und/oder sterilisierende Gut ausgeleitet werden kann. Ist die Ausleitung derart, dass weder pasteurisiertes noch sterilisiertes Gut in den Reinbereich gelangt, kann auf das Stoppen der Zuführung des Guts verzichtet werden.

Sobald nach einem Stoppen der Zuführung des Guts und/oder der Ausleitung der Reinbereich wieder gereinigt ist, kann der Vorrichtung wieder weiteres Gut zugeführt werden bzw. kann die Ausleitung beendet werden.

Im Folgenden wird die Erfindung anhand eines Ausführungsbeispiels und mehrerer Zeichnungen näher erläutert. Dabei zeigen
- Figur 1:: eine schematische Seitenansicht einer erfindungsgemässen Vorrichtung;
- Figur 2:: eine seitliche Ansicht einer Behandlungszone der erfindungsgemässen Vorrichtung;
- Figur 3:: eine perspektivische geschnittene Detailansicht einer Kassette der erfindungsgemässen Vorrichtung;
- Figur 4:: eine perspektivische Ansicht der Behandlungszone der erfindungsgemässen Vorrichtung.

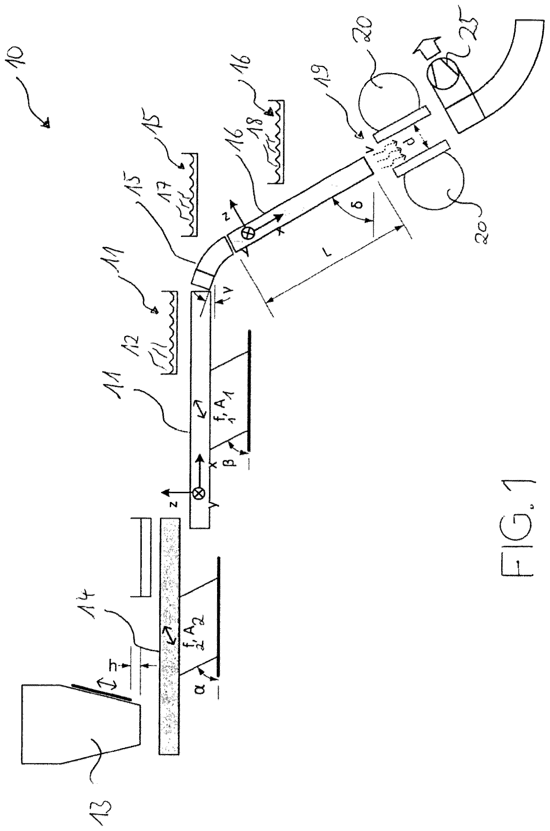

Die in Figur 1 dargestellte Vorrichtung 10 ist zum Pasteurisieren und/oder Sterilisieren von partikelförmigem Gut vorgesehen, wie etwa einem Gewürz, Sesam, Mandeln oder geschälten Pistazien. Sie enthält eine Dosiereinrichtung 13, mit der das Gut auf eine zweite Vibrationsfläche 14 dosiert werden kann. Die zweite Vibrationsfläche 14 ist (bei bestimmungsgemässer Installation der Vorrichtung 10) horizontal ausgerichtet und eben ausgebildet. Sie ist zur Vibrationen mit einer Frequenz f₂ und einer Amplitude A₂, die unter einem Winkel α zur Horizontalen verläuft (bei bestimmungsgemässer Installation der Vorrichtung 10), anregbar. Mit Hilfe dieser zweiten Vibrationsfläche 14 kann der Durchsatz des Guts kontrolliert werden, und es kann auch bereits eine Vorvereinzelung stattfinden.

Stromabwärts von der zweiten Vibrationsfläche 14 enthält die Vorrichtung 10 eine horizontal ausgerichtete erste Vibrationsfläche 11. Diese ist zu Vibrationen mit einer Frequenz f₁ und einer Amplitude A₁, die unter einem Winkel β zur Horizontalen verläuft, anregbar. Hierdurch kann das Gut weiter stromabwärts gefördert und vereinzelt werden. Die erste Amplitude f₁ der ersten Vibrationsfläche 11 ist grösser als die zweite Amplitude f₂ der zweiten Vibrationsfläche 14, was die weitere Vereinzelung begünstigt. Die erste Vibrationsfläche 11 enthält im Gegensatz zur ersten Vibrationsfläche 14 eine Vielzahl von Rinnen 12, in denen das Gut gefördert und mittels deren es vereinzelt werden kann. Diese Rinnen 12 sind in Figur 1 in einem Einschub dargestellt, der die erste Vibrationsfläche 11 in einer Schnittebene senkrecht zur Strömungsrichtung zeigt. Das Profil der Rinnen 12 weist einen geraden mittleren Abschnitt mit einer Breite von 7,5 mm und zwei sich vom mittleren Abschnitt unter einem Winkel von 45° schräg nach oben erstreckende seitliche Abschnitte auf. Die seitlichen Abschnitte benachbarter Rinnen 12 treffen sich an einer Kante. Zwei benachbarte Kanten haben einen Abstand von 16,5 mm.

Stromabwärts von der ersten Vibrationsfläche 11 weist die Vorrichtung 10 eine Umlenkfläche 15 auf. Diese ist derart ausgebildet und angeordnet, dass das Gut darauf umgelenkt und von der ersten Vibrationsfläche 11 zu einer nachfolgend beschriebenen Rutschfläche 16 rutschen kann. Die Umlenkfläche 15 enthält ebenfalls eine Vielzahl von Rinnen 17, welche derart ausgebildet und angeordnet sind, dass das Gut darin rutschen kann. Die Umlenkfläche 15 und ihre Rinnen 17 sind derart auf das Gut und die erste Vibrationsfläche 11 abgestimmt, dass die Partikel des Guts auf einer Parabelbahn im Wesentlichen stromabwärts geführt werden, auf der sie auch allein aufgrund der Einwirkung der Schwerkraft fallen würden. Am stromaufwärtigen Ende der Umlenkfläche 15 hat diese eine anfängliche Neigung γ. Dies erlaubt eine Führung und weitere Vereinzelung der Partikel des Guts. Auch das Profil der in einem weiteren Einschub dargestellten Rinnen 17 weist einen geraden mittleren Abschnitt mit einer Breite von 7,5 mm und zwei sich vom mittleren Abschnitt unter einem Winkel von 45° schräg nach oben erstreckende seitliche Abschnitte auf. Die seitlichen Abschnitte benachbarter Rinnen 17 treffen sich an einer Kante. Zwei benachbarte Kanten haben einen Abstand von 16,5 mm.

Die bereits erwähnte, stromabwärts von der Umlenkfläche 15 angeordnete Rutschfläche 16 ist bezüglich einer Horizontalen unter einem Winkel δ geneigt, der im Falle von Gewürzen vorteilhafterweise 60° beträgt. Auch die Rutschfläche 16 verfügt über eine Vielzahl von Rinnen 18, welche derart ausgebildet und angeordnet sind, dass das Gut darin rutschen kann. Auch das Profil der in einem weiteren Einschub dargestellten Rinnen 18 weist einen geraden mittleren Abschnitt mit einer Breite von 7,5 mm und zwei sich vom mittleren Abschnitt unter einem Winkel von 45° schräg nach oben erstreckende seitliche Abschnitte auf. Die seitlichen Abschnitte benachbarter Rinnen 17 treffen sich an einer Kante. Zwei benachbarte Kanten haben einen Abstand von 16,5 mm.

Noch weiter stromabwärts enthält die Vorrichtung 10 eine Behandlungszone 19. Dort wird das Gut frei fallend mittels eines Elektronenstrahls pasteurisiert und/oder sterilisiert, der von zwei einander gegenüberliegenden Elektronenquellen 20 erzeugt wird.

Die Vorrichtung 10 enthält weiterhin eine Absaugeinrichtung 25, mit dem Prozessgas, welches das Gut umgibt, stromabwärts von der Behandlungszone 19 abgesaugt werden kann.

Zum Pasteurisieren und/oder Sterilisieren von partikelförmigem Gut mit Hilfe dieser Vorrichtung 10 werden die folgenden Schritte durchgeführt:
Mittels der zweiten Vibrationsfläche 14 wird der Durchsatz des Guts kontrolliert, und es findet eine Vorvereinzelung statt. In einem Schritt a) wird das Gut in den Rinnen 12 der ersten Vibrationsfläche 11 gefördert und vereinzelt. Mittels der Elektronenquellen 20 wird in einem Schritt b) ein Elektronenstrahl erzeugt. In einem Schritt c) erfolgt dann ein Pasteurisieren und/oder Sterilisieren des frei fallenden Guts mittels des Elektronenstrahls in der Behandlungszone 19.

Das Gut bewegt sich im Falle von Gewürzen vorteilhaft mit einer Geschwindigkeit von 2,5 m/s durch die Behandlungszone 19. Diese Geschwindigkeit kann durch die Länge und den Neigungswinkel der Rutschfläche 17 eingestellt werden. Die Elektronen des Elektronenstrahls weisen eine Energie auf, die im Bereich von 80 keV bis 300 keV liegt, beispielsweise bei 250 keV. In der Behandlungszone 19 weist der Elektronenstrahl eine mittlere Stromdichte auf, die im Bereich von 10¹⁵ s⁻¹·cm⁻² bis 2,77·10¹⁵ s⁻¹·cm⁻² liegt. Das Gut wird dem Elektronenstrahl für eine Behandlungszeit ausgesetzt, die im Bereich von 5 ms bis 25 ms liegen und beispielsweise 15 ms betragen kann. Hierdurch wird das Gut einer Strahlendosis ausgesetzt, die im Bereich von 1 kGy bis 45 kGy liegen und beispielsweise 12 kGy betragen kann. Das das Gut umgebende Prozessgas wird nach der Pasteurisierung und/oder Sterilisierung in der Behandlungszone 19 mittels der Absaugeinrichtung 25 abgesaugt, und zwar mit einer bevorzugten Absauggeschwindigkeit, die das 1- bis 1,5-fache Geschwindigkeit des Guts während der Pasteurisierung und/oder Sterilisierung beträgt.

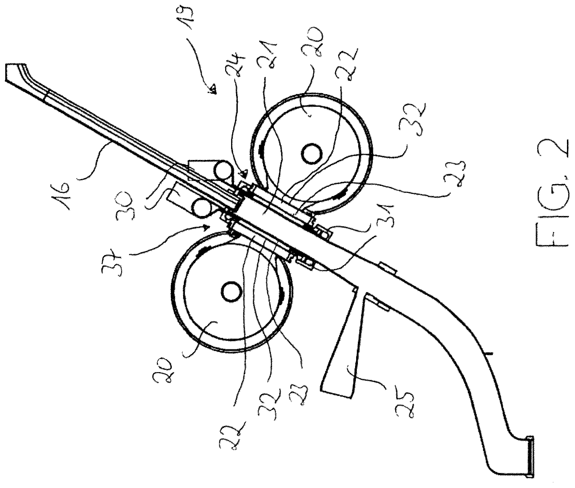

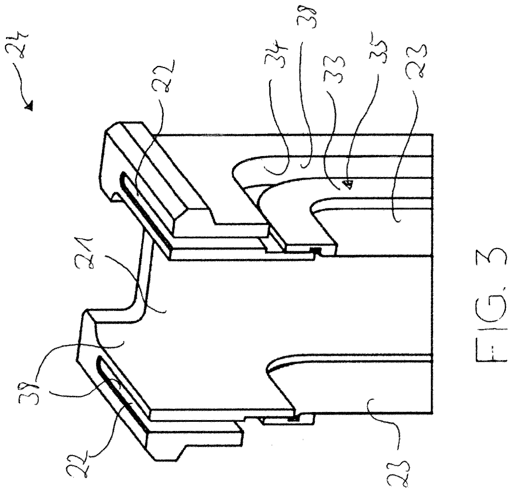

In Figur 2 ist die Behandlungszone 19 in einer Detailansicht dargestellt. Im Bereich der Behandlungszone 19 weist die Vorrichtung 10 eine zwischen Austrittsfenstern 32 der Elektronenquellen 20 angeordnete Kassette 24 auf, von der ein Ausschnitt noch detaillierter in Figur 3 gezeigt ist. Die Kassette 24 ist in einer Kassettenaufnahme 37 eingesetzt. Die Kassette 24 enthält zwei Folienaufnahmen 35 für jeweils eine aus Titan bestehende Schutzfolie 23, die für die Elektronenstrahlen teilweise durchlässig sind. Die Kassette 24 enthält mehrere Begrenzungsflächen 38, die zusammen mit den Schutzfolien 23 einen Gutkanal 21 begrenzen, in dem das Gut mittels der Elektronenstrahlen pasteurisierbar und/oder sterilisierbar ist. Weiterhin enthält die Vorrichtung 10 im Bereich der Behandlungszone 19 zwei Nebenkanäle 22. Diese werden in der in Figur 2 gezeigten Betriebsposition durch Begrenzungsflächen 38 der Kassette 24, die Schutzfolie 23 und die in Figur 3 nicht eingezeichneten Austrittsfenster 32 der Elektronenquellen 20 begrenzt und verlaufen somit zwischen dem Gutkanal 21 und den Elektronenquellen 20. Der Gutkanal 21 ist von den Nebenkanälen 23 fluidgetrennt, und zwar unter anderem durch die Schutzfolie 23.

Durch Eintrittsöffnungen 30 kann Luft eingeführt werden, die die Nebenkanäle 23 parallel zur Strömungsrichtung des Guts durchströmen kann. Stromabwärts kann die Luft aus Austrittsöffnungen 31 wieder austreten. Dieser Luftstrom ermöglicht einerseits das Abführen von Ozon, welches durch die Elektronenstrahlen erzeugt wird, und andererseits eine Kühlung der Elektronenquellen 20 und insbesondere ihrer Austrittsfenster 32.

In Figur 3 ist eine noch detaillierte, geschnittene und perspektivische Ansicht der Kassette 24 gezeigt, in der der Gutkanal 21, die beiden Nebenkanäle 22 und die beiden Schutzfolien 23 erkennbar sind. Der Gutkanal 21 ist durch die beiden Schutzfolien 23 von den Nebenkanälen 22 fluidgetrennt. Die Schutzfolien 23 werden mit Hilfe eines jeweiligen Spannelementes 33 gehalten, die einen Teil der Folienaufnahme 35 bilden. An den dem Gutkanal 21 abgewandten Seiten der Kassette 24 ist jeweils eine Aussparung 34 gebildet, die in der Betriebsposition der Vorrichtung 10 von Austrittsfenstern 32 der Elektronenquellen 20 verschlossen werden und durch die die Elektronenstrahlen hindurchdringen können.

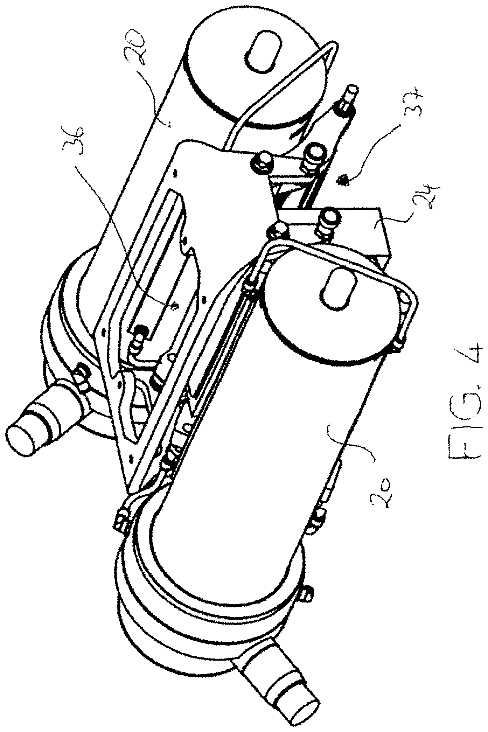

Figur 4 zeigt einen Teil der Behandlungszone 19 mit den Elektronenquellen 20 und der Kassette 24. Das hier nicht dargestellte stromabwärtige Ende der Rutschfläche 17 dringt im montierten Zustand der Vorrichtung 10 in die Öffnung 36 ein und ist mit dem Gutkanal 21 verbunden. Die Elektronenquellen 20 sind derart schwenkbar relativ zur Kassettenaufnahme 37 angeordnet, dass sie von der Kassette 24 weg bewegbar sind. Auf diese Weise ist die Kassette 24 leicht zugänglich, insbesondere wenn die Schutzfolien 23 verschmutzt oder beschädigt sind. Die Schutzfolie 23 kann lösbar von der Folienaufnahme 35 aufgenommen sein.

## Patentansprüche

1. Vorrichtung (10) zum Pasteurisieren und/oder Sterilisieren von partikelförmigem Gut, enthaltend
- mindestens eine Elektronenquelle (20) zum Erzeugen eines Elektronenstrahls,
- eine Behandlungszone (19), in der das Gut insbesondere frei fallend mittels des Elektronenstrahls pasteurisierbar und/oder sterilisierbar ist,
wobei die Vorrichtung (10) im Bereich der Behandlungszone (19) einen Gutkanal (21) aufweist, in dem das Gut mittels des Elektronenstrahls pasteurisierbar und/oder sterilisierbar ist,
**dadurch gekennzeichnet, dass**
die Vorrichtung (10) mindestens einen von einem Fluid durchströmbaren Nebenkanal (22) aufweist, welcher zumindest teilweise zwischen der Elektronenquelle (20) und dem Gutkanal (21) verläuft und vom Gutkanal (21) fluidgetrennt ist,
wobei zwischen der Elektronenquelle (20) und dem Gutkanal (21) eine für den Elektronenstrahl zumindest teilweise durchlässige und insbesondere aus einem Metall, bevorzugt aus Titan, bestehende Schutzfolie (23) angeordnet ist, die den Gutkanal (21) vom Nebenkanal (22) trennt, und wobei die Vorrichtung (10) eine Absaugeinrichtung (25) zum Absaugen von das Gut umgebendem Prozessgas stromabwärts von der Behandlungszone (19) enthält.

2. Vorrichtung (10) gemäss Anspruch 1,
wobei der Nebenkanal (22) zumindest teilweise zwischen der Elektronenquelle (20) und der Schutzfolie (23) angeordnet ist.

3. Vorrichtung (10) gemäss einem der vorangehenden Ansprüche,
wobei die Vorrichtung (10) eine Kassette (24) sowie eine Kassettenaufnahme (37) zum Aufnehmen der Kassette (24) aufweist, in der die Kassette (24) eingesetzt ist, wobei die Kassette (24) zumindest teilweise den Gutkanal (21) und den mindestens einen Nebenkanal (22) begrenzt und eine Folienaufnahme (35) zur Aufnahme der Schutzfolie (23) enthält, wobei die Schutzfolie (23) von der Folienaufnahme (35) aufgenommen ist, und wobei die Elektronenquelle (20) derart beweglich, insbesondere schwenkbar und/oder verschiebbar, relativ zur Kassettenaufnahme (37) angeordnet ist, dass sie von der Kassette (24) weg bewegbar ist.

4. Vorrichtung (10) gemäss einem der vorangehenden Ansprüche,
wobei die Vorrichtung (10) stromabwärts von der Behandlungszone (19) eine Sortiereinrichtung aufweist, welche eine Messeinheit und eine Ausstosseinheit enthält, welche derart ausgebildet sind, dass mittels der Ausstosseinheit einzelne Partikel des Guts anhand mindestens einer mittels der Messeinheit gemessenen Eigenschaft der Partikel ausstossbar sind.

5. Vorrichtung (10) gemäss einem der vorangehenden Ansprüche,
wobei die Vorrichtung (10) mindestens eine stromabwärts von der Behandlungszone (19) angeordnete Gasaustrittsöffnung zum Einblasen eines Reinigungsgases auf das Gut aufweist.

6. Verfahren zum Pasteurisieren und/oder Sterilisieren von partikelförmigem Gut, insbesondere mit einer Vorrichtung (10) gemäss einem der Ansprüche 1 bis 5, enthaltend die folgenden Schritte:
b) Erzeugen eines Elektronenstrahls,
c) Pasteurisieren und/oder Sterilisieren eines, insbesondere frei fallenden, partikelförmigen Guts mittels des Elektronenstrahls in einer Behandlungszone (19),
wobei das Gut im Bereich der Behandlungszone (19) durch einen Gutkanal (21) strömt, in dem das Gut mittels des Elektronenstrahls pasteurisiert und/oder sterilisiert wird,
**dadurch gekennzeichnet, dass**
ein Fluid durch mindestens einen Nebenkanal (22) strömt, welcher zumindest teilweise zwischen der Elektronenquelle (20) und dem Gutkanal (21) verläuft und vom Gutkanal (21) fluidgetrennt ist,
wobei die Schutzfolie (23) den Gutkanal (21) vom Nebenkanal (22) trennt,
und wobei das Gut umgebendes Prozessgas nach der Pasteurisierung und/oder Sterilisierung abgesaugt wird.

7. Verfahren gemäss Anspruch 6,
wobei der Nebenkanal (22) zumindest teilweise zwischen der Elektronenquelle (20) und der Schutzfolie (23) angeordnet ist.

8. Verfahren gemäss der Ansprüche 6 und 7,
wobei sich das Gut mit einer Geschwindigkeit durch die Behandlungszone (19) bewegt, die im Bereich von 1 m/s bis 5 m/s, bevorzugt von 2 m/s bis 4 m/s, besonders bevorzugt von 2 m/s bis 3 m/s liegt.

9. Verfahren gemäss einem der Ansprüche 6 bis 8,
wobei die Elektronen des Elektronenstrahls eine Energie aufweisen, die im Bereich von 80 keV bis 300 keV, bevorzugt von 140 keV bis 280 keV, besonders bevorzugt von 180 keV bis 260 keV liegt.

10. Verfahren gemäss einem der Ansprüche 6 bis 9,
wobei das Gut dem Elektronenstrahl für eine Behandlungszeit ausgesetzt wird, die im Bereich von 5 ms bis 25 ms liegt.

11. Verfahren gemäss einem der Ansprüche 6 bis 10,
wobei das Gut mittels des Elektronenstrahls einer Strahlendosis ausgesetzt wird, die im Bereich von 1 kGy bis 45 kGy, bevorzugt von 8 kGy bis 30 kGy, besonders bevorzugt von 10 kGy bis 16 kGy liegt.

12. Verfahren gemäss einem der Ansprüche 6 bis 11,
der Elektronenstrahl in der Behandlungszone (19) eine mittlere Stromdichte aufweist, die im Bereich von 10¹⁵ s⁻¹·cm⁻² bis 2,77·10¹⁵ s⁻¹·cm⁻² liegt.

13. Verfahren gemäss einem der Ansprüche 6 bis 12,
wobei das Prozessgas mit einer Absauggeschwindigkeit abgesaugt wird, die das 1- bis 3-fache, bevorzugt das 1-bis 1,5-fache der Geschwindigkeit des Guts während der Pasteurisierung und/oder Sterilisierung beträgt.

14. Verfahren gemäss einem der Ansprüche 6 bis 13,
wobei das Gut aus der Gruppe ausgewählt ist, die besteht aus:
- Lebensmitteln, wie beispielsweise Getreide wie etwa Soja, Frühstückscerealien, Snacks, Nüssen wie etwa getrockneten Kokosnüssen, Mandeln, Erdnussbutter, Kakaobohnen, Schokolade, Schokoladenpulver, Schokoladenchips, Kakaoprodukten, Hülsenfrüchten, Kaffee, Samen wie etwa Kürbissamen, Gewürzen (wie beispielsweise Kurkuma, insbesondere in Scheiben), Teemischungen, getrockneten Früchten, Pistazien, trockenen Proteinprodukten, Bäckereiprodukten, Zucker, Kartoffelprodukten, Teigwaren, Babynahrung, getrockneten Eiprodukten, Sojaprodukten wie etwa Sojabohnen, Verdickungsmitteln, Hefen, Hefeextrakten, Gelatine oder Enzymen;
- Tiernahrungsmitteln, wie beispielsweise Pellets, Futter für Wiederkäuer, Geflügel, Wassertiere (insbesondere Fische) oder Haustiere, oder Mischfutter;
- Kunststoff wie etwa PET, beispielsweise in Form von Flocken oder Pellets.

## Claims

1. Apparatus (10) for pasteurizing and/or sterilizing particulate material, comprising
- at least one electron source (20) for generating an electron beam,
- a treatment zone (19) in which the material can be pasteurized and/or sterilized, in particular in a free-falling manner, by means of the electron beam,
wherein the device (10) has a product channel (21) in the region of the treatment zone (19), in which the product can be pasteurized and/or sterilized by means of the electron beam,
**characterized in that**
the device (10) has at least one secondary channel (22) through which a fluid can flow, which runs at least partially between the electron source (20) and the material channel (21) and is fluid-separated from the material channel (21),
wherein a protective foil (23) which is at least partially permeable to the electron beam and in particular consists of a metal, preferably titanium, is arranged between the electron source (20) and the material channel (21) and separates the material channel (21) from the secondary channel (22),
and wherein the device (10) contains an extraction device (25) for extracting process gas surrounding the material downstream of the treatment zone (19).

2. Device (10) according to claim 1,
wherein the secondary channel (22) is arranged at least partially between the electron source (20) and the protective foil (23).

3. Device (10) according to one of the preceding claims, wherein the device (10) has a cassette (24) and a cassette holder (37) for receiving the cassette (24), in which the cassette (24) is inserted, wherein the cassette (24) at least partially delimits the material channel (21) and the at least one secondary channel (22) and contains a foil holder (35) for receiving the protective foil (23), wherein the protective film (23) is received by the film holder (35), and wherein the electron source (20) is arranged movably, in particular pivotably and/or displaceably, relative to the cassette holder (37) in such a way that it can be moved away from the cassette (24) .

4. Device (10) according to one of the preceding claims, wherein the device (10) has a sorting device downstream of the treatment zone (19), which contains a measuring unit and an ejection unit, which are designed in such a way that individual particles of the material can be ejected by means of the ejection unit on the basis of at least one property of the particles measured by means of the measuring unit.

5. Device (10) according to one of the preceding claims, wherein the device (10) has at least one gas outlet opening arranged downstream of the treatment zone (19) for blowing a cleaning gas onto the material.

6. Method for pasteurizing and/or sterilizing particulate material, in particular with an apparatus (10) according to one of claims 1 to 5, comprising the following steps:
b) Generation of an electron beam,
c) and/or sterilization of a particulate product, in particular a free-falling product, by means of the electron beam in a treatment zone (19),
wherein the material flows in the region of the treatment zone (19) through a material channel (21) in which the material is pasteurized and/or sterilized by means of the electron beam,
**characterized in that**
a fluid flows through at least one secondary channel (22), which runs at least partially between the electron source (20) and the material channel (21) and is fluid-separated from the material channel (21),
wherein the protective film (23) separates the material channel (21) from the secondary channel (22),
and wherein process gas surrounding the material is extracted after pasteurization and/or sterilization.

7. Method according to claim 6,
wherein the secondary channel (22) is arranged at least partially between the electron source (20) and the protective film (23).

8. Method according to claims 6 and 7,
wherein the material moves through the treatment zone (19) at a speed which is in the range from 1 m/s to 5 m/s, preferably from 2 m/s to 4 m/s, particularly preferably from 2 m/s to 3 m/s.

9. Method according to any one of claims 6 to 8,
wherein the electrons of the electron beam have an energy which is in the range from 80 keV to 300 keV, preferably from 140 keV to 280 keV, particularly preferably from 180 keV to 260 keV.

10. Method according to any one of claims 6 to 9,
wherein the article exposed to the electron beam for a treatment time which is in the range from 5 ms to 25 ms.

11. Method according to any one of claims 6 to 10,
wherein the material is exposed by means of the electron beam to a radiation dose which is in the range from 1 kGy to 45 kGy, preferably from 8 kGy to 30 kGy, particularly preferably from 10 kGy to 16 kGy.

12. Method according to one of claims 6 to 11,
the electron beam in the treatment zone (19) has an average current density which is in the range from 10¹⁵ s⁻¹·cm⁻² to 2.77·10¹⁵ s⁻¹·cm⁻².

13. Method according to any one of claims 6 to 12,
wherein the process gas is extracted at an extraction speed which is 1 to 3 times, preferably 1 to 1.5 times, the speed of the material during pasteurization and/or sterilization.

14. The method according to any one of claims 6 to 13,
wherein the good is selected from the group consisting of:
- Foods such as cereals such as soy, breakfast cereals, snacks, nuts such as dried coconut, almonds, peanut butter, cocoa beans, chocolate, chocolate powder, chocolate chips, cocoa products, legumes, coffee, seeds such as pumpkin seeds, spices (such as turmeric, especially in slices), tea blends, dried fruits, pistachios, dry protein products, bakery products, sugar, potato products, pasta, baby food, dried egg products, soy products such as soybeans, thickeners, yeasts, yeast extracts, gelatine or enzymes;
- Animal feed, such as pellets, feed for ruminants, poultry, aquatic animals (especially fish) or pets, or compound feed;
- Plastic such as PET, for example in the form of flakes or pellets.

## Revendications

1. Appareil (10) pour la pasteurisation et/ou la stérilisation d'une matière particulaire, comprenant
- au moins une source d'électrons (20) pour générer un faisceau d'électrons,
- une zone de traitement (19) dans laquelle la matière peut être pasteurisée et/ou stérilisée, notamment en chute libre, au moyen du faisceau d'électrons,
dans lequel le dispositif (10) comporte un canal de produit (21) dans la zone de traitement (19), dans lequel le produit peut être pasteurisé et/ou stérilisé au moyen du faisceau d'électrons,
**caractérisé en ce que**
le dispositif (10) comporte au moins un canal secondaire (22) dans lequel un fluide peut s'écouler, qui s'étend au moins partiellement entre la source d'électrons (20) et le canal de matériau (21) et qui est séparé du canal de matériau (21) par un fluide,
dans lequel une feuille de protection (23) au moins partiellement perméable au faisceau d'électrons et notamment constituée d'un métal, de préférence du titane, est disposée entre la source d'électrons (20) et le canal de matériau (21) et sépare le canal de matériau (21) du canal secondaire (22),
et dans lequel le dispositif (10) contient un dispositif d'extraction (25) pour extraire le gaz de traitement entourant le matériau en aval de la zone de traitement (19) .

2. Dispositif (10) selon la revendication 1,
dans lequel le canal secondaire (22) est disposé au moins partiellement entre la source d'électrons (20) et la feuille de protection (23).

3. Dispositif (10) selon l'une des revendications précédentes,
dans lequel le dispositif (10) comporte une cassette (24) et un porte-cassette (37) destiné à recevoir la cassette (24), dans lequel la cassette (24) est insérée, dans lequel la cassette (24) délimite au moins partiellement le canal de matériau (21) et le au moins un canal secondaire (22) et contient un porte-film (35) destiné à recevoir le film protecteur (23), dans lequel le film protecteur (23) est reçu par le support de film (35), et dans lequel la source d'électrons (20) est disposée de manière mobile, en particulier de manière pivotante et/ou déplaçable, par rapport au support de cassette (37) de façon à pouvoir être éloignée de la cassette (24).

4. Dispositif (10) selon l'une des revendications précédentes,
dans lequel le dispositif (10) comporte un dispositif de tri en aval de la zone de traitement (19), qui contient une unité de mesure et une unité d'éjection, conçues de manière à ce que des particules individuelles de la matière puissent être éjectées au moyen de l'unité d'éjection sur la base d'au moins une propriété des particules mesurée au moyen de l'unité de mesure.

5. Dispositif (10) selon l'une des revendications précédentes,
dans lequel le dispositif (10) comporte au moins une ouverture de sortie de gaz disposée en aval de la zone de traitement (19) pour souffler un gaz de nettoyage sur le matériau.

6. Procédé de pasteurisation et/ou de stérilisation de matières particulaires, notamment avec un appareil (10) selon l'une des revendications 1 à 5, comprenant les étapes suivantes :
b) Génération d'un faisceau d'électrons,
c) et/ou la stérilisation d'un produit particulaire, en particulier d'un produit en chute libre, au moyen du faisceau d'électrons dans une zone de traitement (19),
dans lequel la matière circule dans la zone de traitement (19) à travers un canal de matière (21) dans lequel la matière est pasteurisée et/ou stérilisée au moyen du faisceau d'électrons,
**caractérisé par le fait qu'**un fluide circule dans au moins un canal secondaire (22), qui s'étend au moins partiellement entre la source d'électrons (20) et le canal de matériau (21) et est séparé du canal de matériau (21) par un fluide,
dans lequel le film protecteur (23) sépare le canal de matériau (21) du canal secondaire (22),
et dans lequel le gaz de traitement entourant le matériau est extrait après la pasteurisation et/ou la stérilisation.

7. Procédé selon la revendication 6,
dans lequel le canal secondaire (22) est disposé au moins partiellement entre la source d'électrons (20) et le film protecteur (23).

8. Procédé selon les revendications 6 et 7,
dans lequel le matériau traverse la zone de traitement (19) à une vitesse comprise entre 1 m/s et 5 m/s, de préférence entre 2 m/s et 4 m/s, de préférence encore entre 2 m/s et 3 m/s.

9. Procédé selon l'une des revendications 6 à 8,
dans lequel les électrons du faisceau d'électrons ont une énergie comprise entre 80 keV et 300 keV, de préférence entre 140 keV et 280 keV, de préférence encore entre 180 keV et 260 keV.

10. Méthode selon l'une des revendications 6 à 9,
dans laquelle l'article est exposé au faisceau d'électrons pendant une durée de traitement comprise entre 5 ms et 25 ms.

11. Procédé selon l'une des revendications 6 à 10,
dans lequel le
matériau est exposé au moyen du faisceau d'électrons à une dose de rayonnement comprise entre 1 kGy et 45 kGy, de préférence entre 8 kGy et 30 kGy, de préférence encore entre 10 kGy et 16 kGy.

12. Procédé selon l'une des revendications 6 à 11,
le faisceau d'électrons dans la zone de traitement (19) a une densité de courant moyenne qui est comprise entre 10¹⁵ s⁻¹·cm⁻² et 2,77·10¹⁵ s⁻¹·cm⁻².

13. Procédé selon l'une des revendications 6 à 12,
dans lequel le gaz de traitement est extrait à une vitesse d'extraction qui est de 1 à 3 fois, de préférence de 1 à 1,5 fois, la vitesse de la matière pendant la pasteurisation et/ou la stérilisation.

14. La méthode selon l'une des revendications 6 à 13, dans laquelle le produit est choisi dans le groupe constitué par :
- Aliments tels que les céréales telles que le soja, les céréales pour petit-déjeuner, les snacks, les noix telles que la noix de coco séchée, les amandes, le beurre de cacahuète, les fèves de cacao, le chocolat, le chocolat en poudre, les pépites de chocolat, les produits à base de cacao, les légumineuses, le café, les graines telles que les graines de citrouille, les épices (telles que le curcuma), les mélanges de thé, notamment en tranches), mélanges de thé, fruits secs, pistaches, produits protéiques secs, produits de boulangerie, sucre, produits à base de pommes de terre, pâtes, aliments pour bébés, produits à base d'œufs secs, produits à base de soja tels que les graines de soja, épaississants, levures, extraits de levure, gélatine ou enzymes ;
- Aliments pour animaux, tels que les granulés, les aliments pour ruminants, la volaille, les animaux aquatiques (en particulier les poissons) ou les animaux de compagnie, ou les aliments composés ;
- Plastique tel que le PET, par exemple sous forme de flocons ou de granulés.
